(19) Europäisches Patentamt
European Patent Office
Office européen des brevets

(11) Numéro de publication: **0 183 612**
**B1**

(12) # FASCICULE DE BREVET EUROPÉEN

(45) Date de publication du fascicule du brevet: **24.02.88**

(51) Int. Cl.⁴: **C 07 C 149/16**

(21) Numéro de dépôt: **85402262.1**

(22) Date de dépôt: **20.11.85**

(54) **Bis-(perfluoroalkyl-2 éthylthio)-2,2 acétaldéhydes et leur procédé de préparation.**

(30) Priorité: **28.11.84 FR 8418106**

(43) Date de publication de la demande: **04.06.86 Bulletin 86/23**

(45) Mention de la délivrance du brevet: **24.02.88 Bulletin 88/8**

(84) Etats contractants désignés: **AT BE CH DE FR GB IT LI LU NL SE**

(56) Documents cité: **Néant**

(73) Titulaire: **ATOCHEM, 4 & 8, Cours Michelet La Défense 10, F-92800 Puteaux (FR)**

(72) Inventeur: **Bertaina, Brigitte, FLORE JUPITER Place de la Brigue, F-06300 Nice (FR)**
Inventeur: **Cambon, Aimé, 59, avenue du Parc Mosca, F-06000 Nice (FR)**
Inventeur: **Lantz, André, Domane de la Hêtraie, F-69390 Vernaison (FR)**

(74) Mandataire: **Leboulenger, Jean, ATOCHEM Département Propriété Industrielle, F-92091 Paris la Défense 10 Cédex 42 (FR)**

**EP 0 183 612 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1988

**Description**

La présente invention concerne le domaine des produits fluorés obtenus par réaction d'aldéhydes avec les thiols fluorés de formule:

$R_F$-$C_2H_4$-SH (I)

dans laquelle $R_F$ représente une chaîne perfluorée $C_nF_{2n+1}$ linéaire ou ramifiée, n étant un nombre allant de 1 à 20. Ces produits sont notamment intéressants comme intermédiaires pour la fabrication d'agents tensioactifs ou de produits conférant des propriétés hydrophobes et oléophobes aux substrats sur lesquels ils sont appliqués.

La réaction des thiols de formule (I) avec des aldéhydes ou des cétones a déjà été décrite dans différents brevets. Ainsi, le brevet-EP 85 655 décrit la préparation de thioacétals aromatiques par réaction avec un aldéhyde ou une cétone aromatique selon le schéma:

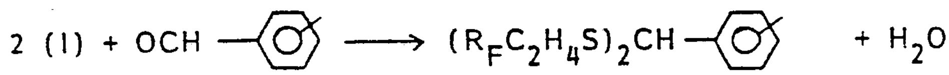

Selon les brevets US-4 239 915 et EP-73 732, d'autres thioacétals sont obtenus à partir de carboxycétones, comme l'acide 0x0-4 pentanoïque, ou de carboxyaldéhydes comme l'acide glyoxylique suivant les schémas:

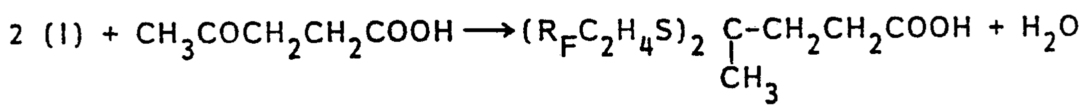

$$2\ (I) + OCH\text{-}COOH \longrightarrow (R_FC_2H_4S)_2CH\text{-}COOH + H_2O$$

Il a maintenant été trouvé qu'avec le glyoxal CHO-CHO une seule fonction aldéhyde réagit et qu'on obtient sélectivement les bis-(perfluoroalkyl-2 éthylthio)-2,2 acétaldéhydes qui forment l'objet de l'invention et répondent à la formule générale:

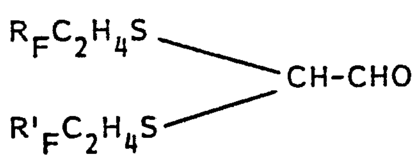
(II)

dans laquelle les symboles $R_F$ et $R'_F$, identiques ou différents, représentent chacun une chaîne perfluorée $C_nF_{2n+1}$ telle que définie précédemment. Contrairement à ce que l'on pouvait craindre, il ne se forme pas ou très peu de double dithioacétal (produit de tétracondensation) ou de double hémithioacétal.

Selon l'invention, on prépare les composés de formule (II) en faisant réagir le glyoxal avec un thiol fluoré de formule (I) ou un mélange de tels thiols, en présence d'un catalyseur acide.

Les perfluoroalkyl-2 éthanethiols de formule (I) sont des produits connus qui peuvent par exemple être obtenus selon les indications du brevet US-3 544 663 par réaction de la thiourée avec un iodure de perfluoroalkyl-2 éthyle ($R_FC_2H_4I$).

Le glyoxal est un produit industriel existant en général sous forme de solution aqueuse à 30 % et un tel produit peut être utilisé tel quel pour le procédé selon l'invention.

Bien que la réaction puisse être effectuée en l'absence de solvant, on opère de préférence en présence d'un solvant qui doit être inerte vis-à-vis des réactifs. Comme exemples de solvants inertes, on peut citer à titre non limitatif les hydrocarbures aliphatiques ou aromatiques tels que le cyclohexane, l'heptane, le benzène ou le toluène, les hydrocarbures halogénés tels que le chlorure de méthylène, le chloroforme, le tétrachlorure de carbone ou le trifluoro-1,1,2 trichloro-1,2,2 éthane, les esters tels que l'acétate d'éthyle, les éthers tels que le diéthyléther, le tétrahydrofuranne ou le dioxanne, et des acides aliphatiques tels que l'acide acétique. La quantité de solvant peut varier dans de larges limites. Elle est généralement comprise entre 0,25 et 2,5 litres par mole de thiol fluoré et, de préférence. entre 0,5 et 1,5 litres.

La réaction est de préférence effectuée à une température comprise entre la température ambiante et 150°C.

La formation du dithioacétal nécessite la présence d'un catalyseur acide tel que, par exemple, l'acide chlorhydrique, l'acide sulfurique, l'acide paratoluènesulfonique, le trifluorure de bore ou le chlorure de zinc. La quantité de catalyseur peut varier dans de larges limites: elle est généralement comprise entre 0,005 et 0,5 mole par mole de thiol, de préférence entre 0,01 et 0,1 mole.

On opère de préférence en engageant une quantité stoechiométrique des deux réactifs, c'est-à-dire deux moles de thiol fluoré par mole de glyoxal, mais il est aussi possible d'utiliser un excés ou un, défaut de thiol par rapport au glyoxal.

La durée de la réaction qui dépend en particulier de la nature du groupe perfluoroalkyle, de la température de réaction, de la nature et de la quantité de solvant et de catalyseur peut varier de quelques dizaines de minutes à plusieurs jours. En fin de réaction, le produit peut être isolé selon des techniques habituelles comme, par exemple, filtration du milieu réactionnel, évaporation des solvants. Si besoin est, le produit de réaction peut être purifié par distillation, recristallisation, lavage, etc...

Dans le procédé selon l'invention, on peut utiliser aussi bien un thiol fluoré pur où le groupe $R_F$ correspond à une chaîne $C_nF_{2n+1}$ dans laquelle n est un nombre entier bien défini (par exemple $R_F = CF_3$, $C_2F_5$, $C_4F_9$, $C_8F_{17}$, etc...) qu'un mélange de thiols fluorés correspondant à différentes valeurs de n. Pour les applications où l'on cherche à mettre à profit les propriétés tensioactives ou les propriétés hydro- et oléophobes de ces dérivés fluorés fonctionnels, on utilise de préférence des produits ou des mélanges de produits où est compris entre 6 et 14.

Les nouveaux composés fluorés répondant à la formule générale (II) sont des auxiliaires précieux qui trouvent des applications dans l'industrie textile et dans celles du cuir et du papier, en particulier pour la fabrication de produits conférant aux textiles, au cuir, au papier ou à d'autres substrats des propriétés aussi bien hydrophobes qu'oléophobes. Ces mêmes composés fluorés sont aussi utilisables pour la préparation de tensioactifs fluorés qui trouvent de nombreuses applications comme agents mouillants, moussants, émulsifiants ou dispersants, comme agents d'étalement des cires, vernis et peinture, comme additifs aux lubrifiants et aux matières plastiques, comme constituants ou additifs des émulseurs synthétiques ou protéiniques utilisés dans la lutte contre l'incendie, et pour la fabrication d'émulsions de carbures fluorés dans l'eau.

Les exemples suivants illustrent l'invention, sans la limiter.

**Exemple 1**

On a chauffé à reflux, à 80°C, sous agitation constante pendant 48 heures un mélange comprenant 28 g de $C_4F_9C_2H_4SH$ (0,1 mole). 9,7 g d'une solution aqueuse de glyoxal à 30 % (0,05 mole), 0,5 g d'acide paratoluènesulfonique et 125 ml de benzène. A la fin de la période de chauffage, le mélange réactionnel a été refroidi à température ambiante, additionné de 300 ml d'eau et extrait avec trois fois 50 ml d'éther éthylique. La phase organique a été séchée sur sulfate de sodium, puis les solvants ont été évaporés et le résidu a été purifié par distillation sous pression réduite. On a ainsi recueilli 22,5 g d'un liquide incolore distillant à 64-66°C sous 1 torr. Ce liquide a été identifié par spectrométrie de masse, RMN et IR comme étant le bis-(tétrahydro-1,1,2,2 perfluorohexylthio)-2,2 acétaldéhyde

$(C_4F_9C_2H_4S)_2$ CH-CHO

Le rendement de la réaction a été de 75 %.

**Analyses élémentaires**

| | C % | H % | S % | F % |
|---|---|---|---|---|
| calculé... | 28 | 1,67 | 10,67 | 57 |
| trouvé..... | 27,2 | 1,8 | 10,8 | 57,5 |

**Exemple 2**

Dans les mêmes conditions que celles utilisées pour l'exemple 1, mais en engageant 38 g (0,1 mole) de $C_6F_{13}C_2H_4SH$ on a obtenu 27,6 g d'un produit distillant à 89-92°C sous 1 torr, identifié comme étant le bis-(tétrahydro-1,1,2,2 perfluoro-octylthiol-2,2 acétaldéhyde:

$(C_6F_{13}C_2H_4S)_2$ CH-CHO

Le rendement de la préparation a été de 69 %.
Le produit est solide à température ambiante et fond à 45-46°C.

**Exemple 3**

Dans les mêmes conditions que celles utilisées pour l'exemple 1, mais en engageant 48 g de $C_8F_{17}C_2H_4SH$ on a obtenu 37 g d'un produit identifié comme étant le bis-(tétrahydro-1,1,2,2 perfluorodécylthio)-2,2 acétaldéhyde. Le point de fusion de ce produit, après recristallisation dans de l'hexane, est de 74-75° C.

**Revendications** pour les Etats contractants: BE, CH, DE, FR, GB, IT, LI, LU, NL, SE

1. Bis-(perfluoroalkyl-2 éthylthio)-2,2 acétaldéhydes répondant à la formule générale:

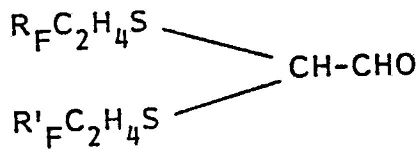

(II)

dans laquelle les symboles $R_F$ et $R_F'$, identiques ou différents, représentent chacun une chaîne perfluorée $C_nF_{2n+1}$, linéaire ou ramifiée, n étant un nombre allant de 1 à 20.

2. Bis-(perfluoroalkyl-2 éthylthio)-2,2 acétaldéhydes selon la revendication 1, dans lesquels n est un nombre allant de 6 à 14.

3. Bis-(perfluoroalkyl-2 éthylthio)-2,2 acétaldéhydes selon la revendication 1 ou 2, dans lesquels $R_F$ et $R_F'$ sont identiques.

4. Mélanges de bis-(perfluoroalkyl-2 éthylthio)-2,2 acétaldéhydes selon l'une des revendications 1 à 3.

5. Procédé de préparation des bis-(perfluroalkyl-2 éthylthio)-2,2 acétaldéhydes selon la revendication 1, caractérisé en ce que l'on fait réagir avec du glyoxal un perfluoroalkyl-2 éthanethiol ou un mélange de tels thiols, en présence d'un catalyseur acide.

6. Procédé selon la revendication 5, dans lequel on opère dans un solvant inerte à une température comprise entre la température ambiante et 150° C.

7. Procédé selon la revendication 5 ou 6, dans lequel le glyoxal est utilisé sous forme de solution aqueuse.

8. Procédé selon l'une des revendications 5 à 7, dans lequel on utilise un thiol ou un mélange de thiols dont la chaîne perfluorée contient de 6 à 14 atomes de carbone.

9. Utilisation des bis-(perfluoroalkyl-2 éthylthio)-2,2 acétaldéhydes selon l'une des revendications 1 à 4 pour la fabrication d'agents tensio-actifs fluorés ou de produits à propriétés hydrophobes et oléophobes.

**Revendications** pour l'Etat contractant: AT

1. Procédé de préparation des bis-(perfluoroalkyl-2 éthylthio)-2,2 acétaldéhydes répondant à la formule générale:

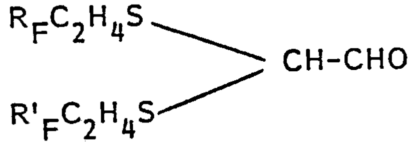

(II)

dans laquelle les symboles $R_F$ et $R_F'$, identiques ou différents, représentent chacun une chaîne perfluorée $C_nF_{2n+1}$, linéaire ou ramifiée, n étant un nombre allant de 1 à 20, caractérisé en ce que l'on fait réagir avec du glyoxal un perfluoroalkyl-2 éthanethiol ou un mélange de tels thiols, en présence d'un catalyseur acide.

2. Procédé selon la revendication 1, dans lequel on opère dans un solvant inerte à une température comprise entre la température ambiante et 150° C.

3. Procédé selon la revendication 1 ou 2, dans lequel le glyoxal est utilisé sous forme de solution aqueuse.

4. Procédé selon l'une des revendications 1 à 3, dans lequel on utilise un thiol ou un mélange de thiols dont la chaîne perfluorée contient de 6 à 14 atomes de carbone.

5. Utilisation des bis-(perfluoroalkyl-2 éthylthio)-2,2 acétaldéhydes selon l'une des revendications 1 à 4 pour la fabrication d'agents tensio-actifs fluorés ou de produits à propriétés hydrophobes et oléophobes.

**Patentansprüche** für die Vertragsstaaten BE, CH, DE, FR, GB, IT, LI, LU, NL, SE

1. Bis-2,2 (2-Perfluoralkylethylthio)-acetaldehyde entsprechend der allgemeinen Formel:

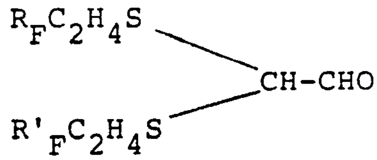

(II)

in der die Reste $R_F$ und $R_F'$ identisch oder verschieden sind und jeweils eine linerare oder verzweigte perfluorierte Kette $C_nF_{2n+1}$ darstellen, wobei n eine Zahl zwischen 1 und 20 ist.

2. Bis-2,2 (2-Perfluoralkylethylthio)-acetaldehyde gemäß Anspruch 1, bei denen n eine Zahl zwischen 6 und 14 ist.

3. Bis-2,2 (2-Perfluoralkylethylthio)-acetaldehyde nach Anspruch 1 oder 2, bei denen $R_F$ und $R_F'$ identisch sind.

4. Gemische von Bis-2,2 (2-Perfluoralkylethylthio)-acetaldehyden gemäß einem der Ansprüche 1 bis 3.

5. Verfahren zur Herstellung der Bis-2,2 (2-Perfluoralkylethylthio)-acetaldehyde gemäß Anspruch 1, dadurch gekennzeichnet, daß man ein 2-Perfluoralkylethanthiol oder ein Gemisch solcher Thiole in Gegenwart eines sauren Katalysators mit Glyoxal reagieren läßt.

6. Verfahren nach Anspruch 5, bei dem man in einem inerten Solvens bei einer Temperatur zwischen der Raumtemperatur und 150° C arbeitet.

7. Verfahren nach Anspruch 5 oder 6, bei dem das Glyoxal in Form einer wässrigen Lösung verwendet wird.

8. Verfahren nach einem der Ansprüche 5 bis 7, bei dem man ein Thiol oder ein Gemisch von Thiolen verwendet, dessen (deren) perfluorierte Kette 6 bis 14 Kohlenstoffatome enthält.

9. Verwendung der Bis-2,2 (2-Perfluoralkylethylthio)-acetaldehyde gemäß einem der Ansprüche 1 bis 4 für die Herstellung von fluorierten oberflächenaktiven Agenzien oder von Produkten mit hydrophoben und oleophoben Eigenschaften.

**Patentansprüche** für den Vertragsstaat: AT

1. Verfahren zur Herstellung von Bis-2,2-(2-Perfluoralkylethylthio)-acetaldehyden gemäß der allgemeinen Formel:

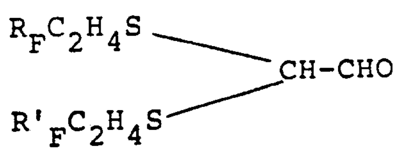

(II)

in der die Reste $R_F$ und $R'_F$ identisch oder verschieden sind und jeweils eine lineare oder verzweigte perfluorierte Kette $C_nF_{2n+1}$ darstellen, wobei n eine Zahl zwischen 1 und 20 ist, dadurch gekennzeichnet, daß man ein 2-Perfluoralkylethanthiol oder ein Gemisch solcher Thiole in Gegenwart eines sauren Katalysators mit Glyoxal reagieren läßt.

2. Verfahren nach Anspruch 1, bei dem man in einem inerten Solvens bei einer Temperatur zwischen der Raumtemperatur und 150° C arbeitet.

3. Verfahren nach Anspruch 1 oder 2, bei dem das Glyoxal in Form einer wässrigen Lösung verwendet wird.

4. Verfahren nach einem der Ansprüche 1 bis 3, bei dem man ein Thiol oder ein Gemisch von Thiolen verwendet, dessen (deren) perfluorierte Kette zwischen 6 und 14 Kohlenstoffatome enthält.

5. Verwendung der Bis-2,2-(2-Perfluoralkylethylthio)-acetaldehyde gemäß einem der Ansprüche 1 bis 4 für die Herstellung von oberflächenaktiven fluorierten Agenzien oder von Produkten mit hydrophoben und oleophoben Eigenschaften.

**Claims** for the contracting states: BE, CH, DE, FR, GB, IT, LI, LU, NL, SE

1. 2,2-Bis (2-perfluoroalkylethylthio) acetaldehydes corresponding to the general formula:

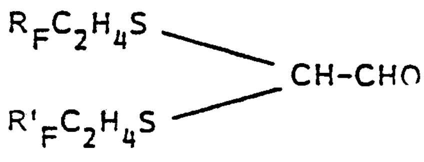

(II)

in which the symbols $R_F$ and $R'_F$, which are identical or different, each denote a linear or branched perfluorinated chain $C_nF_{2n+1}$, n being a number ranging from 1 to 20.

2. 2,2-Bis (2-perfluoroalkylethylthio) acetaldehydes according to Claim 1, in which n is a number ranging from 6 to 14.

3. 2,2-Bis (2-perfluoroalkylethylthio) acetaldehydes according to Claim 1 or 2, in which $R_F$ and $R'_F$ are identical.

4. Mixtures of 2,2-bis (2-perfluoroalkylethylthio) acetaldehydes according to one of Claims 1 to 3.

5. Process for the preparation of 2,2-bis (2-perfluoroalkylethylthio) acetaldehydes according to Claim 1, characterized in that glyoxal is reacted with a 2-perfluoroalkylethanethiol or a mixture of such thiols, in the presence of an acidic catalyst.

6. Process according to Claim 5, in which the operation is performed in an inert solvent at a temperature between ambient temperature and 150° C.

7. Process according to Claim 5 or 6, in which the glyoxal is employed in the form of aqueous solution.

8. Process according to one of Claims 5 to 7, in which a thiol or a mixture of thiols in which the perfluorinated chain contains from 6 to 14 carbon atoms is employed.

9. Use of the 2,2-bis (2-perfluoroalkylethylthio) acetaldehydes according to one of Claims 1 to 4 for the manufacture of fluorinated surface-active agents or of products with hydrophobic andoleophobic properties.

**Claims** for the contracting State: AT

1. Process for the preparation of 2,2-bis (2-perfluoroalkylethylthio) acetaldehydes corresponding to the general formula:

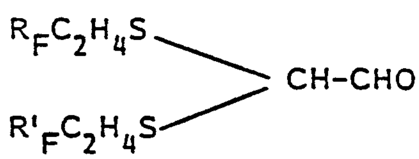

(II)

in which the symbols $R_F$ and $R'_F$, which are identical or different, each denote a linear or branched perfluorinated chain $C_nF_{2n+1}$, n being a number ranging from 1 to 20, characterized in that glyoxal is reacted with a 2-perfluoroalkylethanethiol or a mixture of such thiols, in the presence of an acidic catalyst.

2. Process according to Claim 1, in which the operation is performed in an inert solvent at a temperature between ambient temperature and 150° C.

3. Process according to Claim 1 or 2, in which the glyoxal is employed in the form of aqueous solution.

4. Process according to one of Claims 1 to 3, in which a thiol or a mixture of thiols in which the perfluorinated chain contains from 6 to 14 carbon atoms is employed.

5. Use of the 2,2-bis (2-perfluoroalkylethylthio) acetaldehydes according to one of Claims 1 to 4 for the manufacture of fluorinated surface-active agents or of products with hydrophobic and oleophobic properties.